# EUROPÄISCHE PATENTSCHRIFT

(11) **EP 3 485 311 B1**
(45) Veröffentlichungstag und Bekanntmachung des Hinweises auf die Patenterteilung: **26.02.2020**
(21) Anmeldenummer: 17734726.7
(22) Anmeldetag: 03.07.2017
(51) Int. Cl.: G02B 23/24, A61B 1/00, A61B 1/12, F16K 31/00

(54) **EINRICHTUNG ZUM PRÜFEN DER DICHTIGKEIT EINES CHIRURGISCHEN INSTRUMENTS, AUFBEREITUNGSEINRICHTUNG ZUM AUFBEREITEN VON CHIRURGISCHEN INSTRUMENTEN UND VERWENDUNG EINES QUELLBAREN MATERIALKÖRPERS**
DEVICE FOR TESTING THE LEAKPROOFNESS OF A SURGICAL INSTRUMENT, REPROCESSING DEVICE FOR REPROCESSING SURGICAL INSTRUMENTS AND USE OF A SWELLABLE BODY OF MATERIAL
DISPOSITIF D'ESSAI DE L'ÉTANCHÉITÉ D'UN INSTRUMENT CHIRURGICAL, DISPOSITIF DE PRÉPARATION DESTINÉ À LA PRÉPARATION DES INSTRUMENTS CHIRURGICAUX ET UTILISATION D'UN ÉLÉMENT GONFLABLE

(30) Priorität: 12.07.2016 DE 102016212672
(43) Veröffentlichungstag der Anmeldung: 22.05.2019
(73) Patentinhaber: OLYMPUS Winter & Ibe GmbH, 22045 Hamburg (DE)
(72) Erfinder: LENGSFELD, Michael, 22179 Hamburg (DE); DELZER, Christoph, 21376 Salzhausen (DE); THATE, Henning, 22417 Hamburg (DE); OTTENS, Benjamin, 22309 Hamburg (DE)
(74) Vertreter: Seemann & Partner Patentanwälte mbB
(86) Internationale Anmeldenummer: PCT/EP2017/066426
(87) Internationale Veröffentlichungsnummer: WO 2018/010983

(56) Entgegenhaltungen:
- WO-A1-2015/180951
- GB-A- 476 845
- JP-A- 2009 072 437
- US-A- 2 718 234
- US-A1- 2015 216 608

## Beschreibung

Die Erfindung betrifft eine Einrichtung zum Prüfen der Dichtigkeit eines chirurgischen Instruments, insbesondere Endoskops, mit einer, insbesondere von Luft durchströmbaren, Verbindungseinrichtung zum Verbinden der Einrichtung mit einem Anschluss eines chirurgischen Instruments.

Ferner betrifft die Erfindung eine Aufbereitungseinrichtung zum Aufbereiten von chirurgischen Instrumenten, insbesondere Endoskopen. Außerdem betrifft die Erfindung eine Verwendung eines quellbaren Materialkörpers.

Zur Aufbereitung von, insbesondere flexiblen, Endoskopen werden üblicherweise Reinigungs- und Desinfektionsgeräte (RDG-E) eingesetzt, die auf einem chemisch-thermischen Aufbereitungsprozess basieren. Um die Endoskope durch die Reinigungsflüssigkeit nicht zu beschädigen, ist es wichtig, dass die Hülle des Endoskops vollständig intakt ist und somit Reinigungsflüssigkeit nicht in das Innere des Endoskops eindringen kann. Dies wird im RDG-E vor dem ersten Wassereinlauf überprüft, indem das Endoskop über eine entsprechende Schnittstelle auf einen Überdruck aufgepumpt wird. Anschließend wird der Druckabfall überwacht, wobei aus einem Druckabfall auf eine Leckage in dem Endoskop geschlossen werden kann. Bei fehlgeschlagener Prüfung bzw. zu Prozessende, also zum Ende des Reinigungs- und Desinfektionsprozesses, wird das Endoskop wieder teilweise entlüftet. Die Aufbereitung selbst erfolgt bei geringerem Überdruck im Endoskop.

Entsprechende RDG-E-Systeme der OLYMPUS Winter & Ibe GmbH, Hamburg, sind unter der Bezeichnung "ETD" bekannt. Diese Systeme umfassen einen sogenannten "Leak Tester", der den Dichtigkeitstest durchführt. Dazu wird ein Endoskop initial nach Programmstart zur Dichtigkeitsprüfung mit einem Überdruck von beispielsweise 285 mbar gegenüber dem Umgebungsdruck aufgepumpt und der Überdruck nach dem Dichtigkeitstest auf ca. 150 mbar über Umgebungsdruck reduziert. Dieser Druck wird während der Aufbereitung gehalten und kontinuierlich überwacht, auch um das Eindringen von Wasser von außen zu verhindern.

Bei der Verwendung der Endoskope in der Endoskopie treten vereinzelt Schäden auf, bei denen Endoskope von Biopsiekanälen her punktiert werden. Es entsteht eine Mikroperforation, die bei einem durchgeführten Dichtigkeitstest unter Umständen nicht erkannt wird. Bei der nachfolgenden Aufbereitung wird Spül- und Klarwasser mit einem deutlich größeren Druck als 150 mbar durch die zu spülenden Kanäle geleitet. Dadurch kann dann ein Übertrag von Wasser aus der Kanalspülung in den zu schützenden Bereich des Endoskops erfolgen, was zu einer Druckerhöhung im Endoskop über 150 mbar hinaus führt. Der Leak Tester reagiert darauf mit einem Ablassen von Druckluft aus dem Endoskop, um den Überdruck von 150 mbar zu halten. Dadurch kann Wasser über den Weg der Entlüftung in die Verschlauchung eindringen und schlussendlich in den Leak Tester eingetragen werden und diesen schlimmstenfalls zerstören.

Bei der Prüfung der Dichtigkeit von chirurgischen Instrumenten, z.B. Endoskopen, wird das Endoskop mit einer Dichtigkeitstesteinrichtung verbunden, um zu überprüfen, ob die äußere Hülle des Endoskops sowie die Kanäle des Endoskops vollständig intakt sind.

Vor dem ersten Reinigungsvorgang mit einer Flüssigkeit, wie z.B. Wasser, wird hierbei automatisch überprüft, indem die Dichtigkeitstesteinrichtung über einen entsprechenden Anschluss mit dem Endoskop verbunden wird und anschließend auf einen Überdruck aufgepumpt wird. Danach wird der Druckabfall überwacht, um eine Leckage im Endoskop festzustellen.

Aufgrund der Schnittstelle, die beispielsweise flexible Endoskope haben, um die Dichtigkeit der Endoskophülle zu überprüfen und die Endoskope zu entlüften, besteht die Gefahr, dass bei falscher Handhabung durch einen Anwender Wasser in die intakte Endoskophülle eindringt. Zudem können in den Endoskopkanälen Undichtigkeiten entstehen, die bei dem geringen Prüfdruck im Rahmen der Dichtigkeitsprüfung (ca. 300 mbar) nicht erkannt werden, bei den höheren Spüldrücken (ca. 1300 mbar) jedoch dafür sorgen, dass Wasser in die Endoskophülle eindringt, wodurch auch der Überdruck in der Endoskophülle (ca. 150 mbar) nicht verhindert werden kann. Dies kann dazu führen, dass das Endoskop beschädigt wird.

Sofern in einem undichten Endoskop Wasser vorhanden ist, kann dies dazu führen, dass das eingedrungene Wasser beim Entlüften in das Reinigungs- und Desinfektionsgerät (RDG-E) gelangen kann, wodurch dieses ebenfalls beschädigt wird.

Die Prüfung chirurgischer Instrumente auf Dichtigkeit sowie Einrichtungen dafür sind hinlänglich bekannt. Dokument JP 2009 072437 zeigt ein Beispiel mit den Merkmalen des Oberbegriffs des Anspruchs 1. Selbsttätige Ventile mit quellbaren Materialkörpern sind z.B. aus dem Bereich der Heizungs- und Lüftungstechnik bekannt, siehe Dokumente GB 476 845 und US 2 718 234 als Beispiel.

Ausgehend von diesem Stand der Technik besteht die Aufgabe der Erfindung darin, die Prüfung der Dichtigkeit von chirurgischen Instrumenten, insbesondere Endoskopen, auf einfache und ggf. kostengünstige Weise zu verbessern.

Gelöst wird diese Aufgabe durch eine Einrichtung zum Prüfen der Dichtigkeit eines chirurgischen Instruments, insbesondere Endoskops, mit einer, insbesondere von Luft durchströmbaren, Verbindungseinrichtung zum Verbinden der Einrichtung mit einem Anschluss eines chirurgischen Instruments, die dadurch weitergebildet wird, dass die Verbindungseinrichtung ein selbsttätiges Ventil mit einem quellbaren Materialkörper aufweist.

Die Erfindung beruht auf den Gedanken, dass bei einer Dichtigkeitsprüfeinrichtung für ein chirurgisches Instrument die Verbindungseinrichtung von Luft bei der Entlüftung des Instruments durchströmbar ist, wobei die Verbindungseinrichtung die Dichtigkeitsprüfeinrichtung und ein Endoskop miteinander verbindet. Hierbei ist in der von Luft durchströmbaren Verbindungseinrichtung ein selbsttätiges Ventil angeordnet, wobei mittels des durch Wasser quellbaren Materialkörpers in der Verbindungseinrichtung erkannt wird, ob Wasser in der Luftströmung aus dem chirurgischen Instrument vorhanden ist. Durch den Kontakt mit Wasser in der ausströmenden Luft aus dem chirurgischen Instrument quillt hygroskopisch der Materialkörper, wodurch aufgrund des Kontakts mit Wasser der Kanal der Verbindungseinrichtung mittels des ausgequollenen Materialkörpers verstopft bzw. gesperrt wird, wodurch die Luftströmung durch die Verbindungseinrichtung unterbrochen wird.

Dadurch, dass mittels des quellbaren Materialkörpers auf einfache Weise das Vorhandensein von Wasser in der Luftströmung erkannt wird und nach Quellung des Materialkörpers der Kanal in der Verbindungseinrichtung gesperrt ist, wird verhindert, dass in das chirurgische Instrument unbemerkt eingedrungenes Wasser in das Reinigungs- und Desinfektionsgerät migriert, wodurch hierdurch das Reinigungs- und Desinfektionsgerät sowie weitere Endoskope nicht beschädigt werden.

Dazu ist weiterhin vorgesehen, dass der quellbare Materialkörper eingerichtet ist, um bei Kontakt mit einer Flüssigkeit, insbesondere Wasser, zu quellen und den Durchfluss von Luft, insbesondere bei der Entlüftung, durch die Verbindungseinrichtung zu sperren. Hierdurch wird eine Ventilfunktion erreicht, wobei bei Kontakt des Materialkörpers mit Wasser aus dem Endoskop der Materialkörper zum Quellen gebracht wird und dadurch die Verbindungseinrichtung in Durchflussrichtung des Luftstroms bei Entlüftung absperrt.

Insbesondere ist hierzu die Verbindungseinrichtung mit einem Kanal ausgebildet, der von Luft durchströmbar ist, wobei in dem Kanal der quellbare Materialkörper angeordnet ist. Dabei ist der Materialkörper vorzugsweise im Kanal austauschbar angeordnet.

Gemäß einer bevorzugten Ausführungsform ist weiterhin bei der Einrichtung vorgesehen, dass der quellbare Materialkörper hygroskopisch ist und/oder der quellbare Materialkörper aus Zellulose, insbesondere faserverstärkter Zellulose, hergestellt ist und/oder dass der quellbare Materialkörper als ein quellbarer Membrankörper ausgebildet ist.

Insbesondere ist der Materialkörper als Quellscheibe in einer Ausführungsform ausgebildet.

Vorzugsweise ist der quellbare Materialkörper in einem von Luft durchströmbaren Gehäuse mit einer Lufteintrittsseite und einer Luftaustrittsseite angeordnet.

Des Weiteren ist es hierbei bevorzugt, dass an der Lufteintrittsseite des Gehäuses ein Sieb und/oder an der Luftaustrittsseite des Gehäuses ein Sieb angeordnet sind, wodurch der Materialkörper zwischen der Lufteintrittsseite und der Luftaustrittsseite angeordnet ist. Durch die Verwendung von Sieben an der Lufteintrittsseite und der Luftaustrittsseite wird der quellbare Materialkörper begrenzt, wobei durch die Siebe der Materialkörper kompakt zusammengehalten wird und ein Austreten des Materials des quellbaren Materialkörpers verhindert wird.

Außerdem ist es in einer Ausgestaltung der Einrichtung vorgesehen, dass die Verbindungseinrichtung als eine Verbindungsleitung, insbesondere Entlüftungsleitung, oder als ein Adapter ausgebildet ist. Hierbei ist z. B. eine Verbindungsleitung erfindungsgemäß mit dem quellbaren Materialkörper, insbesondere einer Quellscheibe, versehen. Darüber hinaus ist es in einer Alternative möglich, dass der quellbare Materialkörper in dem mit einem Kanal versehenen Adapter angeordnet ist.

Ferner zeichnet sich eine Ausführungsform der Einrichtung dadurch aus, dass die Verbindungseinrichtung einen Flüssigkeitssensor, insbesondere einen Wassersensor, aufweist, wodurch die Erkennung von Wasser im zu prüfenden bzw. geprüften chirurgischen Instrument erhöht wird.

Dazu ist weiterhin vorgesehen, dass der Flüssigkeitssensor bezogen auf die Strömungsrichtung der Luft bei der Entlüftung durch die Verbindungseinrichtung eingangsseitig vor dem quellbaren Materialkörper angeordnet ist. Mittels des Flüssigkeitssensors wird z. B. der Widerstand der Luft bzw. des kreisförmigen Mediums überwacht. Fällt der Widerstand aufgrund von Kontakt mit Wasser aus der Luft ab, wird ein Signal ausgelöst, so dass festgestellt werden kann, dass die Sperrung der Verbindungseinrichtung aufgrund des durch Wasser gequollenen Materialkörper hierdurch erfolgt.

Darüber hinaus wird die Aufgabe gelöst durch eine Aufbereitungseinrichtung zum Aufbereiten von chirurgischen Instrumenten, insbesondere Endoskopen, mit einer voranstehenden beschriebenen Einrichtung zum Prüfen der Dichtigkeit eines chirurgischen Instruments. Zur Vermeidung von Wiederholungen wird auf die obigen Ausführungen ausdrücklich verwiesen.

Ferner wird die Erfindung gelöst durch eine Verwendung eines quellbaren Materialkörpers in einer Einrichtung zum Prüfen der Dichtigkeit eines chirurgischen Instruments, insbesondere Endoskop, wie sie voranstehend beschrieben ist. Hierzu wird auf die obigen Ausführungen ebenfalls ausdrücklich verwiesen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines Reinigungs- und Desinfektionsgeräts;
- Fig. 2: einen Querschnitt durch einen Druckluftschlauch zur Verbindung einer Druckluftquelle mit einem Endoskop und
- Fig. 3: schematisch einen Querschnitt durch einen Druckluftschlauch gemäß einer weiteren Ausführungsform.

In den Zeichnungen sind jeweils gleiche oder gleichartige Elemente und/oder Teile mit denselben Bezugsziffern versehen, so dass von einer erneuten Vorstellung jeweils abgesehen wird.

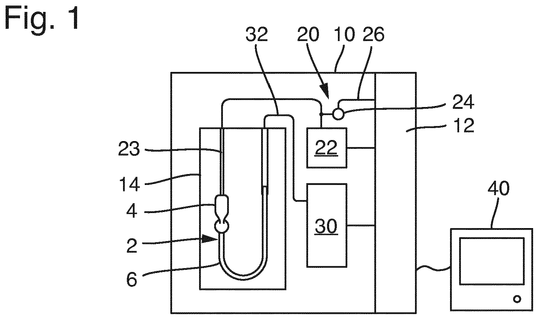

Fig. 1 zeigt eine schematische Darstellung eines Aufbereitungssystems 10, in dessen Reinigungskammer 14 ein flexibles Endoskop 2 angeordnet ist, das bereit ist, gereinigt und desinfiziert zu werden. Das Endoskop 2 umfasst einen Griff 4 sowie einen flexiblen Schaft 6, die beide an Anschlüsse des Aufbereitungssystems 10 angeschlossen sind.

Der Schaft 6 des Endoskops 2 ist über einen Anschluss mit einer Aufbereitungsvorrichtung 30 des Aufbereitungssystems 10 verbunden, während der Handgriff 4 über einen Druckluftschlauch 23 mit einer Druckluftquelle 22, beispielsweise einem Kompressor, mit der Vorrichtung 20 zur Druckluftbeaufschlagung, kurz "Leakage Tester" genannt, verbunden ist. Diese ist, ebenso wie die Aufbereitungsvorrichtung 30, mit einer Steuervorrichtung 12 des Aufbereitungssystems 10 verbunden, die somit auch eine Steuervorrichtung für die Vorrichtung 20 zur Druckluftbeaufschlagung darstellt.

Ausgangs der Druckluftquelle 22 ist ein Luftdrucksensor 24 an den Druckluftschlauch 23 angeschlossen, der den Überdruck der Luft in dem Druckluftschlauch 23 über dem Umgebungsdruck misst und entsprechende Signale über eine Signalleitung 26 ebenfalls an die Steuervorrichtung 12 des Aufbereitungssystems 10 übermittelt. Die Steuervorrichtung 12 ist außerdem mit einer Anzeigevorrichtung 40 verbunden, mittels der die Steuervorrichtung 12 von außen beeinflusst werden kann und umgekehrt Daten aus der Steuervorrichtung 12, beispielsweise Überdruckmessdaten von dem Luftdrucksensor 24 und/oder Prozessdaten, angezeigt werden können.

Die Vorrichtung 20 zur Druckluftbeaufschlagung ist mit einer Druckluftquelle 22 über einen Druckluftschlauch 23 mit einem nur schematisch angedeuteten Endoskop 2 verbunden, wodurch Druckluft in das Endoskop 2 gepumpt wird und danach auch wieder ausgelassen wird.

Mit dem Druckluftschlauch 23 ist ein Luftdrucksensor 24 verbunden, der den Druck der Druckluft im Druckluftschlauch 23 und im Endoskop 2 misst. Der Luftdrucksensor 24 kann alternativ auch am Endoskop 2 direkt messen oder am Ausgang der Druckluftquelle 22.

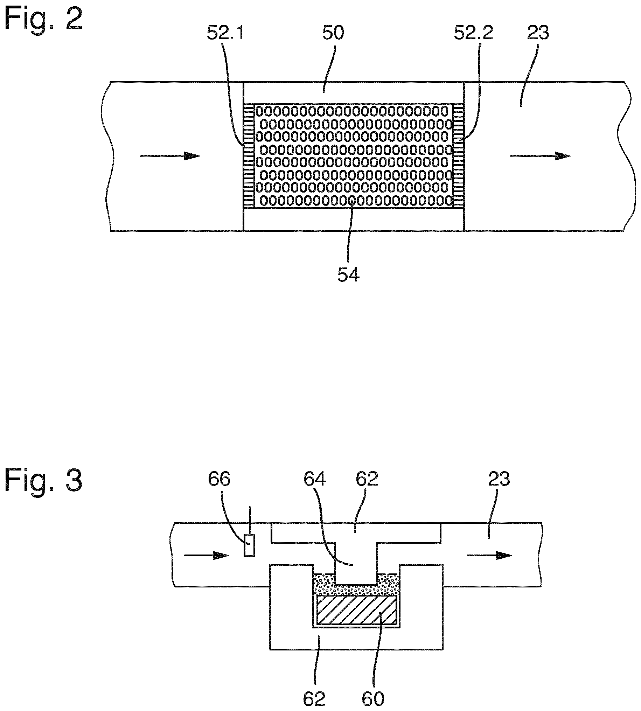

In den Fig. 2 und 3 sind jeweils verschiedene Ausführungsformen eines Druckluftschlauches 23 im Querschnitt schematisch gezeigt.

Bei dem in Fig. 2 gezeigten Druckluftschlauch 23 ist im Druckluftschlauch 23 eine Patrone 50 angeordnet, die beidseits zum Durchlasse eines Luftstroms ein Sieb 52.1 und ein Sieb 52.2 aufweist. Bei Durchführung der Dichtigkeitsprüfung des Endoskops beim Ablassen der in das Endoskop gepumpten Luft ist das Sieb 52.1 an der Lufteintrittsseite und das Sieb 52.2 an der Luftaustrittsseite angeordnet. Zwischen den Sieben 52.1, 52.2 ist ein Quellmaterial bzw. ein quellbarer Materialkörper 54 angeordnet, der Luft aus dem Endoskop beim Durchströmen von trockener Luft bzw. beim Ablassen der Luft durchlässt.

Bei Kontakt des Quellmaterials 54 mit Wasser im Luftstrom quillt der Materialkörper 54 und sperrt den Durchluftschlauch 23 ab, so dass der Luftstrom unterbrochen wird und blockiert wird. Hierdurch wird bei der Quellung des Quellkörpers 54 eine komplette Blockade bzw. Sperre im Druckluftschlauch 23 erreicht. Dadurch wird eine Kontamination von in weiteren Endoskopen mit Wasser bei der Reinigung von mehreren Endoskopen in dem Aufbereitungssystem vermieden. Nach Kontakt des Quellmaterials bzw. des Materialkörpers 54 mit Wasser, erfolgt ein Austausch der Patrone 50 im Druckluftschlauch 23.

Anstelle des quellbaren Materialkörpers 54 kann die Patrone 50 so auch quellbares Membranmaterial bzw. eine quellbare Membran aufweisen, die bei Kontakt mit Wasser quillt und ebenfalls zu einer Blockade der Luftströmung im Druckluftschlauch 23 führt.

Bei dem in Fig. 3 dargestellten Ausführungsbeispiel ist in einer topfartigen Vertiefung eines Ventilkörpers 62 eine Quellscheibe 60 angeordnet. Bei der Entlüftung des Endoskops und der Überprüfung der Dichtigkeit wird der Luftstrom mäanderförmig durch den Ventilkörper 62 hindurchgeführt, in dem die Quellscheibe 60 angeordnet ist. Enthält der durch den Ventilkörper 62 durchgeführte Luftstrom Wasser, so quillt die Quellscheibe 60 auf und gerät in Kontakt mit einem Vorsprung 64 des Ventilkörpers 62, so dass die Quellscheibe im aufgequollenen Zustand den Vorsprung 64 umgibt und den Luftstrom unterbricht. Hierdurch wird die freie Fließstrecke des Volumenstroms bei der Entlüftung unterbrochen und die Austrittsseite des Ventilkörpers 62 von der Eintrittsseite für den Luftstrom unterbrochen. Der aufgequollene Zustand der Quellscheibe 60 ist gestrichelt dargestellt.

In einer weiteren Ausgestaltung kann zusätzlich bzw. optional an der Eintrittsseite der Luftströmung ein Wassersensor 66 angeordnet sein, mittels dem zusätzlich erkannt wird, ob Wasser in dem entlüftenden Luftstrom vorhanden ist. Hierdurch kann in der Aufbereitungseinrichtung zusätzlich erkannt werden, dass die Quellscheibe 60 aufgrund von Wasser im Endoskop aufgequollen ist.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Endoskop
- 4: Griff
- 6: flexibler Schaft
- 10: Aufbereitungssystem
- 12: Steuereinheit
- 14: Reinigungskammer
- 20: Vorrichtung zur Druckluftbeaufschlagung
- 22: Druckluftquelle
- 23: Druckluftschlauch
- 24: Luftdrucksensor
- 26: Signalleitung
- 30: Aufbereitungsvorrichtung
- 32: Spülschlauch
- 40: Anzeigeeinheit
- 50: Patrone
- 52.1, 52.2: Sieb
- 54: Materialkörper
- 60: Quellscheibe
- 62: Ventilkörper
- 64: Vorsprung
- 66: Wassersensor

## Patentansprüche

1. Einrichtung zum Prüfen der Dichtigkeit eines chirurgischen Instruments (2), insbesondere Endoskop (2), mit einer, insbesondere von Luft durchströmbaren, Verbindungseinrichtung (23) zum Verbinden der Einrichtung mit einem Anschluss eines chirurgischen Instruments (2), **dadurch gekennzeichnet, dass** die Verbindungseinrichtung (23) ein selbsttätiges Ventil mit einem quellbaren Materialkörper (54, 60) aufweist.

2. Einrichtung nach Anspruch 1, **dadurch gekennzeichnet, dass** der quellbare Materialkörper (54, 60) eingerichtet ist, um bei Kontakt mit einer Flüssigkeit, insbesondere Wasser, zu quellen und den Durchfluss von Luft durch die Verbindungseinrichtung (23) zu sperren.

3. Einrichtung nach Anspruch 1 oder 2, **dadurch gekennzeichnet, dass** der quellbare Materialkörper (54, 60) hygroskopisch ist und/oder der quellbare Materialkörper (54, 60) aus Zellulose, insbesondere faserverstärkter Zellulose, hergestellt ist und/oder dass der quellbare Materialkörper (54, 60) als ein quellbarer Membrankörper ausgebildet ist.

4. Einrichtung nach einem der Ansprüche 1 bis 3, **dadurch gekennzeichnet, dass** der quellbare Materialkörper (54, 60) in einem von Luft durchströmbaren Gehäuse mit einer Lufteintrittsseite und einer Luftaustrittsseite angeordnet ist.

5. Einrichtung nach Anspruch 4, **dadurch gekennzeichnet, dass** an der Lufteintrittsseite des Gehäuses ein Sieb und/oder an der Luftaustrittsseite des Gehäuses ein Sieb angeordnet sind.

6. Einrichtung nach einem der Ansprüche 1 bis 5, **dadurch gekennzeichnet, dass** die Verbindungseinrichtung (23) als eine Verbindungsleitung, insbesondere Entlüftungsleitung, oder als ein Adapter ausgebildet ist.

7. Einrichtung nach einem der Ansprüche 1 bis 6, **dadurch gekennzeichnet, dass** die Verbindungseinrichtung (23) einen Flüssigkeitssensor, insbesondere einen Wassersensor, aufweist.

8. Einrichtung nach Anspruch 7, **dadurch gekennzeichnet, dass** der Flüssigkeitssensor bezogen auf die Strömungsrichtung der Luft durch die Verbindungseinrichtung (23) eingangsseitig vor dem quellbaren Materialkörper (54, 60) angeordnet ist.

9. Aufbereitungseinrichtung (10) zum Aufbereiten von chirurgischen Instrumenten (2), insbesondere Endoskopen (2), mit einer Einrichtung zum Prüfen der Dichtigkeit eines chirurgischen Instruments (2), insbesondere Endoskop (2), nach einem der Ansprüche 1 bis 8.

10. Verwendung eines quellbaren Materialkörpers (54, 60) in einer Einrichtung zum Prüfen der Dichtigkeit eines chirurgischen Instruments (2), insbesondere Endoskop (2), nach einem der Ansprüche 1 bis 8.

## Claims

1. A device for testing the leakproofness of a surgical instrument (2), in particular an endoscope (2), having a connecting device (23), through which air in particular can flow, for connecting the device to a port of a surgical instrument (2), **characterized in that** the connecting device (23) has a self-actuating valve with a swellable body of material (54, 60).

2. The device according to Claim 1, **characterized in that** the swellable body of material (54, 60) is configured to swell upon contact with a liquid, in particular water, and to block the flow of air through the connecting device (23).

3. The device according to Claim 1 or 2, **characterized in that** the swellable body of material (54, 60) is hygroscopic and/or the swellable body of material (54, 60) is produced from cellulose, in particular fiber-reinforced cellulose, and/or the swellable body of material (54, 60) is designed as a swellable membrane body.

4. The device according to any one of Claims 1 to 3, **characterized in that** the swellable body of material (54, 60) is arranged in a housing, through which air can flow, having an air inlet side and an air outlet side.

5. The device according to Claim 4, **characterized in that** a sieve is arranged at the air inlet side of the housing and/or a sieve is arranged at the air outlet side of the housing.

6. The device according to any one of Claims 1 to 5, **characterized in that** the connecting device (23) is configured as a connecting line, in particular a ventilation line, or as an adapter.

7. The device according to any one of Claims 1 to 6, **characterized in that** the connecting device (23) has a liquid sensor, in particular a water sensor.

8. The device according to Claim 7, **characterized in that** the liquid sensor is arranged at the inlet side before the swellable body of material (54, 60) relative to the flow direction of the air through the connecting device (23).

9. A reprocessing device (10) for reprocessing surgical instruments (2), in particular endoscopes (2), having a device for testing the leakproofness of a surgical instrument (2), in particular an endoscope (2), according to any one of Claims 1 to 8.

10. Use of a swellable body of material (54, 60) in a device for testing the leakproofness of a surgical instrument (2), in particular an endoscope (2), according to any one of Claims 1 to 8.

## Revendications

1. Dispositif pour contrôler l'étanchéité d'un instrument chirurgical (2), en particulier d'un endoscope (2), avec un dispositif de raccordement (23) apte à être traversé en particulier par de l'air, pour le raccordement du dispositif à un raccord d'un instrument chirurgical (2), **caractérisé en ce que** le dispositif de raccordement (23) présente une soupape automatique avec un corps en matériau gonflable (54, 60).

2. Dispositif selon la revendication 1, **caractérisé en ce que** le corps en matériau gonflable (54, 60) est agencé pour gonfler au contact d'un liquide, en particulier de l'eau, et pour bloquer le flux d'air à travers le dispositif de raccordement (23).

3. Dispositif selon la revendication 1 ou la revendication 2, **caractérisé en ce que** le corps en matériau gonflable (54, 60) est hygroscopique et/ou le corps en matériau gonflable (54, 60) est en cellulose, en particulier en cellulose renforcée par des fibres, et/ou **en ce que** le corps en matériau gonflable (54, 60) est réalisé sous la forme d'un corps de membrane gonflable.

4. Dispositif selon l'une des revendications 1 à 3, **caractérisé en ce que** le corps en matériau gonflable (54, 60) est disposé dans un boîtier pouvant être traversé par de l'air et qui présente un côté d'entrée d'air et un côté de sortie d'air.

5. Dispositif selon la revendication 4, **caractérisé en ce qu'**un écran est agencé sur le côté d'entrée d'air du boîtier et/ou un écran est agencé sur le côté de sortie d'air du boîtier.

6. Dispositif selon l'une quelconque des revendications 1 à 5, **caractérisé en ce que** le dispositif de raccordement (23) est conçu comme une conduite de raccordement, en particulier une conduite de circulation d'air, ou comme un adaptateur.

7. Dispositif selon l'une des revendications 1 à 6, **caractérisé en ce que** le dispositif de raccordement (23) comprend un capteur de liquide, en particulier un capteur d'eau.

8. Dispositif selon la revendication 7, **caractérisé en ce que** le capteur de liquide est agencé du côté de l'entrée devant le corps de matière gonflable (54, 60) par rapport au sens d'écoulement de l'air à travers le dispositif de raccordement (23).

9. Dispositif de préparation (10) pour la préparation d'instruments chirurgicaux (2), en particulier d'endoscopes (2), avec un dispositif pour contrôler l'étanchéité d'un instrument chirurgical (2), en particulier d'un endoscope (2), selon l'une des revendications 1 à 8.

10. Utilisation d'un corps en matériau gonflable (54, 60) dans un dispositif pour contrôler l'étanchéité d'un instrument chirurgical (2), en particulier d'un endoscope (2), selon l'une des revendications 1 à 8.
